# EUROPÄISCHE PATENTSCHRIFT

(11) **EP 3 387 996 B1**
(45) Veröffentlichungstag und Bekanntmachung des Hinweises auf die Patenterteilung: **17.07.2019**
(21) Anmeldenummer: 17173452.8
(22) Anmeldetag: 30.05.2017
(51) Int. Cl.: A61B 6/03, A61B 6/00, G01T 1/24, G01R 27/16, H01L 21/66

(54) **ÜBERWACHUNG DER FUNKTIONSFÄHIGKEIT VON INTEGRIERTEN SCHALTUNGEN**
MONITORING OF THE OPERATION OF INTEGRATED CIRCUITS
SURVEILLANCE DE LA CAPACITÉ DE FONCTIONNEMENT DE CIRCUITS INTÉGRÉS

(43) Veröffentlichungstag der Anmeldung: 17.10.2018
(73) Patentinhaber: Siemens Healthcare GmbH, 91052 Erlangen (DE)
(72) Erfinder: Hosemann, Michael, 91056 Erlangen (DE)

(56) Entgegenhaltungen:
- DE-A1-102012 112 981
- DE-A1-102015 213 911
- US-A1- 2014 246 705

## Beschreibung

Die Erfindung betrifft eine integrierte Schaltung mit einer Leckstromüberwachungsfunktion für Durchkontaktierungen im Substrat der integrierten Schaltung. Überdies betrifft die Erfindung einen Röntgendetektor. Außerdem betrifft die Erfindung ein Röntgensystem. Weiterhin betrifft die Erfindung ein Verfahren zum Überwachen von Leckströmen in einer integrierten Schaltung.

Mit Hilfe moderner bildgebender Verfahren werden häufig zwei- oder dreidimensionale Bilddaten erzeugt, die zur Visualisierung eines abgebildeten Untersuchungsobjekts und darüber hinaus auch für weitere Anwendungen genutzt werden können.

Häufig basieren die bildgebenden Verfahren auf der Erfassung von Röntgenstrahlung, wobei sogenannte Projektionsmessdaten erzeugt werden. Beispielsweise können Projektionsmessdaten mit Hilfe eines Computertomographie-Systems, auch CT-System genannt, oder eines C-Bogensystems akquiriert werden. Bei CT-Systemen läuft gewöhnlich eine an einer Gantry angeordnete Kombination aus Röntgenquelle und gegenüberliegend angeordnetem Röntgendetektor um einen Messraum um, in dem sich das Untersuchungsobjekt (das im Folgenden ohne Beschränkung der Allgemeinheit als Patient bezeichnet wird) befindet. Das Drehzentrum (auch "Isozentrum" genannt) fällt dabei mit einer sogenannten Systemachse z zusammen. Bei einem oder mehreren Umläufen wird der Patient mit Röntgenstrahlung der Röntgenquelle durchstrahlt, wobei mit Hilfe des gegenüberliegenden Röntgendetektors Projektionsmessdaten bzw. Röntgenprojektionsdaten erfasst werden.

Die bei der CT-Bildgebung verwendeten Röntgendetektoren weisen gewöhnlich eine Mehrzahl an Detektionseinheiten auf, die meist in Form eines regelmäßigen Pixelarrays angeordnet sind. Die Detektionseinheiten erzeugen jeweils für auf die Detektionseinheiten auftreffende Röntgenstrahlung ein Detektionssignal, welches zu bestimmten Zeitpunkten hinsichtlich Intensität und spektraler Verteilung der Röntgenstrahlung analysiert wird, um Rückschlüsse auf das Untersuchungsobjekt zu erhalten und Projektionsmessdaten zu erzeugen.

Die Röntgendetektoren weisen üblicherweise eine Schicht aus Detektormaterial auf. Weiterhin ist in und auf einem Siliziumsubstrat eine Schicht mit analogen und digitalen integrierten Ausleseschaltkreisen angeordnet. Entfernt von den Ausleseschaltkreisen befinden sich platzverbrauchende elektrische Einheiten, wie zum Beispiel eine Spannungsversorgungschaltung und eine Spannungsmesseinrichtung. Diese können zum Beispiel auf einer von dem Siliziumsubstrat getrennten, über die Durchkontaktierungen mit den Ausleseschaltkreisen elektrisch verbundenen Leiterplatte angeordnet sein. Diese größer dimensionierten Schaltungseinrichtungen sind also von den Ausleseschaltkreisen räumlich separiert angeordnet. Bei einer herkömmlichen Verdrahtung würde ein Schaltkreis durch Bondpads auf der Ober-(Schaltungs-)Seite an allen vier Außenkanten kontaktiert werden. Rings um diese ist noch ein Bondrahmen im Gehäuse erforderlich. Diese beiden Teile erfordern viel Platz zwischen Pixelschaltungen benachbarter Ausleseschaltkreise, was zu einem Dosisverlust führt.

Vorteilhafterweise können bei einer Verdrahtung mit Durchkontaktierungen einzelne Pixel näher beieinander positioniert werden, wodurch aufgrund der größeren Pixelfläche der Dosisverlust verringert wird bzw. ein verbessertes Signal-/Rauschverhältnis erzielt werden kann.

Zwischen den Ausleseschaltkreisen sowie den elektrischen Einheiten sind sogenannte Durchkontaktierungen, im Englischen auch als Through-Silicon Via, abgekürzt TSV, bezeichnet, ausgebildet, welche durch das Siliziumsubstrat hindurch verlaufen und die genannten Einheiten elektrisch verbinden.

Durch mechanische und thermische Beanspruchungen und Umwelteinflüsse können um die Durchkontaktierungen herum angeordnete Isolationsschichten schleichend ihre Isolationseigenschaften verlieren und dadurch Leckströme in das Siliziumsubstrat des Röntgendetektors entstehen lassen. Diese Leckströme verändern die elektrischen Eigenschaften der elektronischen Schaltkreise des Röntgendetektors und können damit die Bildqualität eines Röntgendetektors negativ beeinflussen.

Durchkontaktierungen sind eine neue Technologie, an die Röntgendetektoren hohe Zuverlässigkeitsanforderungen stellen. Eine Möglichkeit der Gewährleistung der Zuverlässigkeit dieser Bauelemente besteht darin, die Durchkontaktierungen durch aufwändige Tests hinsichtlich ihrer Zuverlässigkeit in Röntgendetektoren zu qualifizieren. Trotzdem wäre es wünschenswert, die Isolationseigenschaften der Durchkontaktierungen auch während der Anwendung der Röntgendetektoren zu überwachen, um deren Qualität zu gewährleisten. Zudem könnte man dadurch auch weitere Erfahrungen hinsichtlich der Zuverlässigkeit dieser Technologie sammeln und auf Basis dieser Erfahrungen zukünftige Testverfahren verbessern.

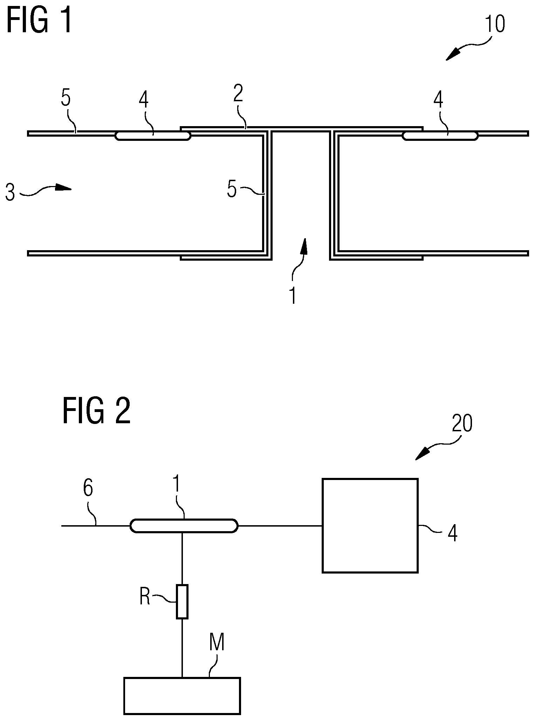

Die für das Auslesen und Ansteuern der Röntgendetektoren verwendeten Schaltkreise sind üblicherweise an separate Versorgungsspannungseinheiten und separate Massebereiche für analoge und digitale Schaltungsteile angeschlossen. Die elektrische Verbindung zwischen diesen Bauelementen erfolgt durch die beschriebenen Durchkontaktierungen. Diese Durchkontaktierungen bestehen aus einer Leitbahn und einer Isolationsschicht, die die Leitbahn gegen das ebenfalls leitende Silizium des Substrats isoliert. Wird diese Isolationsschicht beschädigt, so entsteht daraus bei anliegender Versorgungsspannung ein Leckstrom, der unkontrolliert in andere Schaltungsteile fließt und damit die Schaltungen stören kann. Der grundlegende Aufbau eines integrierten Schaltkreises eines Röntgendetektors mit Durchkontaktierung ist in FIG 1 veranschaulicht. Ein entsprechendes Schaltbild einer Durchkontaktierung für eine aktive Schaltung eines integrierten Schaltkreises ist in FIG 2 gezeigt.

Die Leckströme betragen typischerweise wenige Nano- bis Mikroampere. Sie sind daher nicht direkt über die Überwachung der Stromaufnahme der Schaltkreise des Röntgendetektors messbar, da die typische Stromaufnahme dieser Schaltkreise im Betrieb viele Milliampere beträgt und zusätzlich Schwankungen unterliegt.

In US 2014/ 0 246 705 A1 wird ein Testverfahren zum Testen der Funktionsfähigkeit von Durchkontaktierungen von elektronischen Schaltungen beschrieben. Im Rahmen des Testverfahrens wird in einem Testmodus an eine im Normalbetrieb mit einer funktionellen elektronischen Schaltungseinheit verbundene Durchkontaktierung eine Testspannung angelegt und eine an einem Überwachungsknoten anliegende, von dem Isolationszustand der Durchkontaktierung abhängige elektrische Spannung mit einer Referenzspannung verglichen.

In DE 10 2012 112 981 A1 ist eine Fotodiodenanordnung beschrieben, welche eine Siliziumscheibe mit einer ersten Oberfläche und einer zweiten Oberfläche aufweist. Die Fotodiodenanordnung weist eine Vielzahl wiederaufgefüllter leitender Wege durch die Siliziumscheibe auf, welche eine Dotierungsart aufweisen, die sich von der Dotierungsart des Substrates unterscheidet. Dabei bildet eine Grenzfläche zwischen den wiederaufgefüllten leitenden Wegen und dem Substrat Diodenübergänge. Die Fotodiodenanordnung weist eine gemusterte dotierte Schicht auf der ersten Oberfläche auf, die die wiederaufgefüllten leitenden Wege überlappt, wobei die gemusterte dotierte Schicht eine Anordnung von Fotodioden definiert.

In DE 10 2015 213 911 A1 wird ein Verfahren zum Erzeugen eines Röntgenbildes und eine Datenverarbeitungseinrichtung zur Ausführung des Verfahrens beschrieben.

Es ist somit eine Aufgabe der vorliegenden Erfindung, eine Schaltungsanordnung mit einer Leckstromüberwachungsfunktion für Durchkontaktierungen im Substrat einer integrierten Schaltung anzugeben, mit der die Leckströme überwacht werden können.

Diese Aufgabe wird durch eine integrierte Schaltung gemäß Patentanspruch 1, einen Röntgendetektor gemäß Patentanspruch 10, ein Röntgensystem gemäß Patentanspruch 11 und ein Verfahren zum Überwachen von Leckströmen in einer integrierten Schaltung gemäß Patentanspruch 12 gelöst.

Die erfindungsgemäße integrierte Schaltung, vorzugsweise eine Detektorschaltung, mit Leckstromüberwachungsfunktion für Durchkontaktierungen im Substrat der integrierten Schaltung, weist eine aktive Schaltung und mindestens einen Schaltungsanschluss, beispielsweise einen Signalkontakt, einen Masseanschluss oder einen Anschluss für eine Betriebsspannung, welcher mit der aktiven Schaltung über eine Durchkontaktierung elektrisch verbunden ist, auf. Zudem umfasst die integrierte Schaltung einen offenen Signalkontakt und eine ausschließlich Überwachungszwecken dienende Überwachungsdurchkontaktierung. Überdies weist die integrierte Schaltung eine Messschaltung mit einem Messeingang und einem Referenzspannungseingang auf. Der offene Signalkontakt ist über die Überwachungsdurchkontaktierung mit dem Messeingang der Messschaltung elektrisch verbunden. Die Überwachungsdurchkontaktierung dient also nicht der Verbindung von Funktionselementen mit einem Signaleingang oder Signalausgang. Vielmehr stellt die Überwachungsdurchkontaktierung eine zusätzliche Durchkontaktierung dar, deren Isolation über die Messschaltung überwacht wird. Da die Durchkontaktierungen geometrisch regelmäßig angeordnet sein sollten, sind typischerweise ohnehin ungenutzte Durchkontaktierungen vorhanden, von denen eine oder mehrere erfindungsgemäß zur Überwachung der Funktionsfähigkeit der integrierten Schaltung genutzt werden. Tritt ein Defekt der Isolation der Überwachungsdurchkontaktierung auf, so wird dieser Vorgang mit Hilfe der Messschaltung detektiert und der Defekt der Überwachungsdurchkontaktierung wird als repräsentativ für einen Defekt der übrigen Durchkontaktierungen in der integrierten Schaltung aufgefasst. Die integrierte Schaltung kann auch eine Auswerteschaltung umfassen, welche ein von der Messschaltung ausgegebenes Messergebnis dahingehend auswertet, ob ein Defekt der Überwachungsdurchkontaktierung aufgetreten ist, und eine entsprechende Meldung an den Benutzer ausgibt.

Teil der integrierten Schaltung ist auch ein Betriebsspannungskontakt zur Spannungsversorgung der integrierten Schaltung. Der Betriebsspannungskontakt ist über einen Vorwiderstand elektrisch mit dem Messeingang verbunden. Die Detektion eines Leckstroms erfolgt durch Vergleich einer an dem Messeingang der Messschaltung liegenden elektrischen Spannung mit einer an dem Referenzspannungseingang liegenden elektrischen Spannung. Bei dem Vergleich wird ein Spannungsabfall über den genannten Vorwiderstand gemessen. Der Vorwiderstand kann zum Beispiel einen ohmschen Widerstand, beispielsweise aus Polysilizium, umfassen. Der Vorwiderstand kann auch als separate aktive Schaltung implementiert sein.

Eine solche aktive Schaltung wäre zum Beispiel eine Stromquelle, deren Strom so dimensioniert ist, dass über den intakten Isolationswiderstand der zu überwachenden Durchkontaktierung eine elektrische Spannung zwischen circa 10% und 90% der Betriebsspannung abfällt. Eine aktive Schaltung kann vorteilhaft platzsparender dimensioniert werden als ein durch Polysilizium ausgestalteter ohmscher Widerstand.

Die integrierte Schaltung kann als aktive Schaltung Photosensorchips oder Speicherschaltkreise umfassen. Die aktive Schaltung kann insbesondere für den Fall, dass es sich bei der integrierten Schaltung um eine Detektorschaltung handelt, Ausleseschaltkreise aufweisen.

Indem die Leckströme selbst und nicht eine Veränderung an der Versorgungsspannung oder der Stromstärke der Stromversorgung gemessen werden, kann die Überwachung auf einen relativ kleinen Messbereich beschränkt werden, was die Überwachung vereinfacht und eine verbesserte Genauigkeit ermöglicht. Beispielsweise lässt sich die Überwachung der Durchkontaktierung durch die Nutzung einfacher, kostengünstiger Bauteile realisieren. Denn die Schaltung kann einfach gestaltet sein, da der Vorwiderstand selbst bei kleinen Leckströmen einen großen Spannungsabfall erzeugt. Dadurch ist das Signal/Rauschverhältnis in jedem Fall ausreichend groß, so dass auch eine einfache Schwellwertschaltung verwendet werden kann.

Durch die ständige Überwachung der Durchkontaktierungen wird eine frühzeitige Erkennung von sich anbahnenden Defekten ermöglicht und auffällige Bildartefakte können auf diese Weise verhindert werden. Weiterhin erlaubt die in die integrierte Schaltung integrierte Messschaltung auch eine vereinfachte und automatisierte Überwachung der Isolation der Durchkontaktierungen im Anwendungsfall.

In diesem Zusammenhang soll noch erwähnt werden, dass unter den Begriff Durchkontaktierung auch Anordnungen fallen sollen, bei denen mehrere Überwachungsdurchkontaktierungen in einem Schaltkreis verteilt sind und entweder durch zusätzliche Messschaltungen überwacht werden oder durch ein- und dieselbe Messschaltung überwacht werden. Bei letzterer Anordnungsweise können ohne zusätzlichen Aufwand mehrere Durchkontaktierungen mit Hilfe einer einzigen Spannungsmessschaltung überwacht werden. Insbesondere bei einer sehr hohen Anzahl von Überwachungsdurchkontaktierungen kann eine Überwachung auf eine bestimmte Anzahl von Spannungsmessungen begrenzt sein. Indem die parallel geschalteten Durchkontaktierungen durch einen einzigen Überwachungsvorgang getestet werden, können im Vergleich zu einer Überwachung einzelner Durchkontaktierungen zum Beispiel in einem Zeitintervall mehr Durchkontaktierungen überwacht bzw. getestet werden, so dass eine höhere Wahrscheinlichkeit einer Erkennung einer Isolationsschädigung besteht.

Der erfindungsgemäße Röntgendetektor weist ein Substrat auf und eine Sensorschicht auf dem Substrat. Weiterhin umfasst der erfindungsgemäße Röntgendetektor eine erfindungsgemäße integrierte Schaltung, wobei die Durchkontaktierungen der integrierten Schaltung an Stellen ohne aktive Schaltung durch das Substrat verlaufen und die aktiven Schaltungen über die normalen Verdrahtungslagen kontaktieren. Das Substrat umfasst üblicherweise Halbleitermaterial, wie zum Beispiel Silizium. Halbleitermaterialien haben eine gewisse elektrische Leitfähigkeit. Zudem nimmt ihre Leitfähigkeit mit der Temperatur zu, so dass insbesondere bei einer betriebsbedingten oder umgebungsbedingten Erwärmung des Röntgendetektors Leckströme durch das Substrat fließen können und zum Beispiel Schaltkreise in Ihrer Funktion beeinträchtigen oder beeinflussen können. Mit Hilfe der erfindungsgemäßen integrierten Schaltung wird eine Überwachung solcher durch Defekte von Isolationen bedingter Leckströme auch im regulären Betrieb erreicht, so dass eine erhöhte Zuverlässigkeit des Detektors erzielt wird.

Das erfindungsgemäße Röntgensystem, vorzugsweise ein Computertomographiesystem, weist eine Scaneinheit mit mindestens einem Detektor mit einer Mehrzahl von Röntgendetektoreinheiten auf. Zudem umfasst das erfindungsgemäße Röntgensystem eine Steuereinrichtung zum Ansteuern der Scaneinheit. Mindestens eine der Röntgendetektoreinheiten umfasst eine erfindungsgemäße integrierte Schaltung.

Das erfindungsgemäße Verfahren wird zum Überwachen von Leckströmen in einer integrierten Schaltung, vorzugsweise einer Detektorschaltung, mit einem erfindungsgemäßen Aufbau angewandt. Bei dem Verfahren wird eine an dem Messeingang der Messschaltung anliegende Messspannung gemessen. Zudem wird eine an dem Referenzspannungseingang anliegende Referenzspannung gemessen. Weiterhin wird ein Vergleich auf Basis der beiden gemessenen Spannungswerte durchgeführt und es wird auf Basis des Vergleichs ermittelt, ob die Überwachungsdurchkontaktierung defekt ist. Das Verfahren teilt die Vorteile der erfindungsgemäßen integrierten Schaltung.

Einzelne Komponenten der erfindungsgemäßen integrierten Schaltung können zum überwiegenden Teil in Form von Softwarekomponenten ausgebildet sein. Dies betrifft insbesondere Teile der Messschaltung und eine eventuell vorhandene Auswerteeinrichtung zur Auswertung der erfassten Messgrößen. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können benötigte Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher verwendete integrierte Schaltungen nach einer entsprechenden Modifikation der Hardware auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer integrierten Schaltung ladbar ist, mit Programmabschnitten, um alle Schritte des erfindungsgemäßen Verfahrens auszuführen, wenn das Programm in der integrierten Schaltung ausgeführt wird. Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen

Zum Transport zu der integrierten Schaltung und/oder zur Speicherung an oder in der integrierten Schaltung kann ein computerlesbares Medium, beispielsweise ein Memorystick, eine Festplatte oder ein sonstiger transportabler oder fest eingebauter Datenträger dienen, auf welchem die von einem Chip oder einer Rechnereinheit der integrierten Schaltung einlesbaren und ausführbaren Programmabschnitte des Computerprogramms gespeichert sind. Der Chip oder die Rechnereinheit können z.B. hierzu einen oder mehrere zusammenarbeitende Mikroprozessoren oder dergleichen, wie zum Beispiel ein Mikrokontroller, aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren Beschreibungsteilen weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung der erfindungsgemäßen integrierten Schaltung ist der Betriebsspannungskontakt direkt mit dem Referenzspannungseingang der Messschaltung verbunden. D.h., die Betriebsspannung liegt am Referenzspannungseingang der Messschaltung an. Sie kann direkt als Referenzspannung für einen Vergleich mit der Messspannung genutzt werden oder über einen Spannungsteiler entsprechend reduziert als Referenzspannung für diesen Vergleich genutzt werden. Vorteilhaft wird bei dieser Ausgestaltung keine separate Referenzspannungsversorgung benötigt, so dass die integrierte Schaltung einfach und platzsparend aufgebaut sein kann.

In einer Variante der erfindungsgemäßen integrierten Schaltung ist der Referenzspannungseingang der Messschaltung mit einem separaten Referenzspannungskontakt elektrisch verbunden. Bei dieser Variante liegt an dem Referenzspannungseingang nicht die Betriebsspannung an, sondern eine gesondert für die Referenzspannung erzeugte elektrische Spannung. Diese kann für den Fall einer separaten Referenzspannungsquelle zum Beispiel höher als die Betriebsspannung sein. Für diesen Fall wird die Referenzspannung über einen Spannungsteiler auf ein niedrigeres Niveau, beispielsweise unter das Niveau der Betriebsspannung reduziert, um sie mit der Messspannung vergleichen zu können.

Die zu überwachende Spannung über dem Isolationswiderstand der Überwachungsdurchkontaktierung wird durch den Vorwiderstand typischerweise zwischen 10% und 90% der Betriebsspannung liegen. Die Referenzspannung sollte etwas darüber liegen. Die Vergleichsschaltung bzw. Messschaltung spricht dann auf ein Unterschreiten der Referenzspannung durch die überwachte Spannung, d.h. die Messspannung an. Vorteilhaft kann man durch eine separate Referenzspannung eine höhere Spannung anlegen. Dadurch werden Leckströme leichter detektierbar, insbesondere bei höheren Isolationswiderstandswerten.

In einer besonders vorteilhaften Variante der erfindungsgemäßen integrierten Schaltung ist die Messschaltung dazu eingerichtet, eine an dem Messeingang anliegende elektrische Messspannung mit einer Referenzspannung zu vergleichen und ein Vergleichsergebnis auszugeben. Auf Basis des Vergleichsergebnisses kann ermittelt werden, wie hoch ein Spannungsabfall an dem Vorwiderstand der integrierten Schaltung ausfällt. Diese Information kann dazu genutzt werden, zu ermitteln, ob die Überwachungsdurchkontaktierung defekt ist, d.h. ein Leckstrom von der Überwachungsdurchkontaktierung nach Masse fließt.

In einer ebenfalls sehr vorteilhaften Ausgestaltung der erfindungsgemäßen integrierten Schaltung ist diese dazu eingerichtet, auf Basis des Vergleichsergebnisses zu ermitteln, ob die Überwachungsdurchkontaktierung defekt ist. Hierfür kann die die Messschaltung der integrierten Schaltung dazu eingerichtet sein, eine Spannungsdifferenz zwischen der Referenzspannung und der Messspannung zu ermitteln. Es wird dann auf Basis davon, ob die ermittelte Spannungsdifferenz einen Schwellwert überschritten hat, ermittelt, ob die Überwachungskontaktierung defekt ist. Hierfür wird der Vorwiderstand so gewählt, dass bei einem Unterschreiten eines vorbestimmten Wertes des Isolationswiderstands der Überwachungsdurchkontaktierung ein signifikanter Spannungsabfall an dem Vorwiderstand entsteht, der den genannten Schwellwert überschreitet.

Wie bereits erwähnt, kann die erfindungsgemäße integrierte Schaltung auch eine Mehrzahl von Überwachungskontaktierungen aufweisen, welche über die integrierte Schaltung verteilt sind. Auf diese Weise kann die statistische Wahrscheinlichkeit, mittels des Defekts einer Überwachungsdurchkontaktierung auf defekte Durchkontaktierungen zu schließen, verbessert werden. Die Überwachungsdurchkontaktierungen können durch unabhängige Messschaltungen oder auch ein und dieselbe Messschaltung mit Hilfe eines Zeitmultiplexverfahrens überwacht werden. Es können auch einfach mehrere Überwachungsdurchkontaktierungen parallel an eine Messschaltung geschaltet werden. Der benötigte Vorwiderstand verringert sich dann. Es ist dann zwar nicht möglich zu detektieren, welche Überwachungsdurchkontaktierung defekt ist, der Defekt wird aber erkannt.

Die Erfindung wird im Folgenden unter Hinweis auf die beigefügten Figuren anhand von Ausführungsbeispielen noch einmal näher erläutert. Es zeigen:
FIG 1 einen Ausschnitt des Querschnitts eines herkömmlichen integrierten Schaltkreises mit Durchkontaktierungen,
FIG 2 einen Schaltplan eines herkömmlichen integrierten Schaltkreises mit einer Darstellung der aktiven Schaltung und eines Isolationswiderstands einer Durchkontaktierung,
FIG 3 eine vereinfachte Schaltskizze einer Messanordnung einer integrierten elektronischen Schaltung gemäß einem Ausführungsbeispiel der Erfindung,
FIG 4 ein Computertomographiesystem mit einem Detektor mit einer Detektorschaltung mit einer Detektorleckstrom-Überwachung gemäß einem Ausführungsbeispiel der Erfindung.

In FIG 1 ist eine Detektorschaltung 10 eines Röntgendetektors gezeigt, welche eine Durchkontaktierung 1 umfasst, die über Verdrahtungen 2 mit aktiven Schaltungen 4 elektrisch verbunden ist. Die aktiven Schaltungen 4 sind auf der Oberseite eines die Detektorschaltung 10 tragenden Substrats 3 angeordnet und können zum Beispiel Teil eines integrierten Ausleseschaltkreises zum Auslesen von im Detektormaterial (nicht gezeigt) des betreffenden Röntgendetektors erzeugten Detektionssignalen sein. Getrennt von den integrierten Ausleseschaltkreisen befindet sich das Detektormaterial (nicht gezeigt), mit dem Röntgenstrahlen empfangen und in elektrische Signale gewandelt werden. Die Schaltungen 4 sind mit Anschlüssen (Signalanschlüssen, Spannungsversorgungsanschlüssen) elektrisch verbunden, die sich aus Platzgründen, d.h., um möglichst viel Detektorfläche zu erhalten, auf der der Oberseite gegenüberliegenden Unterseite des Substrats 3 befinden.

Um eine elektrische Verbindung zwischen den aktiven Schaltungen 4 und den auf der Unterseite befindlichen Kontakten (nicht gezeigt) zu erhalten, sind durch das Siliziumsubstrat 3 Durchkontaktierungen 1 geführt.

Dabei kann jede dieser Durchkontaktierungen auch aus mehreren einzelnen, elektrisch parallel geschalteten Durchkontaktierungen bestehen.

Durchkontaktierungen haben an Ihrer Außenseite Isolationsschichten 5, die diese gegen das Siliziumsubstrat 3 isolieren. Wie bereits erwähnt, können durch mechanische und thermische Beanspruchungen und Umwelteinflüsse die verwendeten Durchkontaktierungen 1 schleichend ihre Isolationseigenschaften verlieren und dadurch Leckströme in das Substrat 3 fließen. Diese Leckströme verändern die elektrischen Eigenschaften der aktiven Schaltungen 4 und können damit die Bildqualität einer Abbildung durch einen von der Detektorschaltung 10 angesteuerten Detektor negativ beeinflussen.

In FIG 2 ist eine schematische Darstellung 20 eines Teils einer integrierten Schaltung eines Röntgendetektors gezeigt. Eine aktive Schaltung 4 ist über eine elektrische Durchkontaktierung 1 mit einem Schaltungsanschluss, in diesem Fall ein Signalkontakt 6, elektrisch verbunden. Die Durchkontaktierung 1 ist durch einen Isolationswiderstand R gegen das Substrat bzw. die Masse M der integrierten Schaltung isoliert.

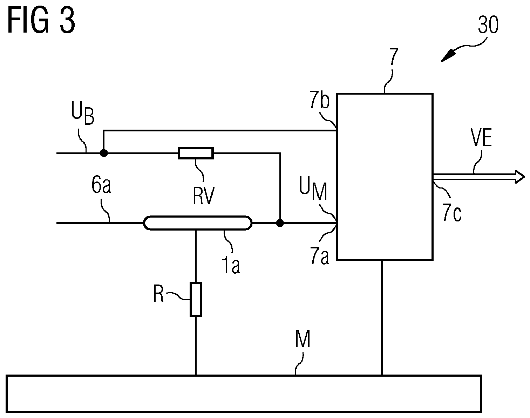

In FIG 3 ist ein Schaltplan einer als ASIC-Schaltung bzw. integrierte Schaltung ausgebildeten Detektorschaltung 30 mit einer Leckstromüberwachung einer Durchkontaktierung gemäß einem Ausführungsbeispiel der Erfindung gezeigt.

Die Detektorschaltung 30 umfasst einen offenen Signalkontakt 6a, der über eine Überwachungsdurchkontaktierung 1a mit einem Messeingang 7a einer Messschaltung 7 elektrisch verbunden ist. Die Messschaltung 7 weist zudem einen Referenzspannungseingang 7b auf, der an einen Betriebsspannungskontakt U_{B} angeschlossen ist, der der Spannungsversorgung der integrierten Schaltung 30 dient. Die Betriebsspannung U_{B} liegt zudem an einem Vorwiderstand RV an, der zwischen dem Betriebsspannungskontakt U_{B}, und dem Messeingang 7a der Messschaltung 7 liegt. Die Überwachungsdurchkontaktierung 1a weist einen Isolationswiderstand R gegen das die Masse M bildende Substrat auf. Weiterhin umfasst die Detektorschaltung 30 auch aktive Schaltungen (nicht gezeigt) sowie Durchkontaktierungen zwischen den aktiven Schaltungen und zugehörigen Signalanschlüssen (nicht gezeigt). Die Überwachung auf Leckströme funktioniert nun derart, dass ein Vergleich auf Basis der an dem Messeingang 7a liegenden Messspannung U_{M} und der Betriebsspannung U_{B} durchgeführt wird. Fällt an dem Vorwiderstand RV eine elektrische Spannung ab, die einen vorbestimmten Schwellwert überschreitet, so wird dies durch die Messschaltung 7 als Hinweis darauf gewertet, dass der Isolationswiderstand R der Überwachungsdurchkontaktierung 1a defekt ist. Der Schwellwert kann zum Beispiel durch eine auf Basis der Betriebsspannung erzeugte Komparatorspannung erzeugt werden, mit der die Messspannung U_{M} verglichen wird. Der Zustand der Überwachungsdurchkontaktierung 1a wird nun als repräsentativ für den Zustand der übrigen Durchkontaktierungen (nicht gezeigt) angesehen. Ein entsprechendes Vergleichsergebnis VE wird von der Messschaltung 7 über einen Ausgang 7c an einen Benutzer übermittelt. Mithin wird eine Meldung über einen Defekt bzw. das Auftreten von Leckströmen ausgegeben. Daraufhin kann ein Austausch der Detektorschaltung 30 vorgenommen werden.

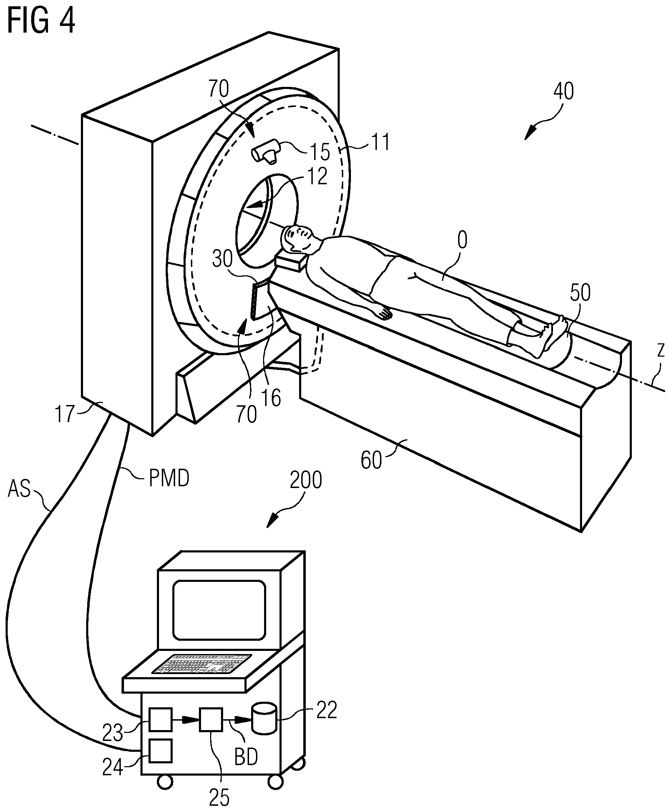

In FIG 4 ist ein Computertomographiesystem 40 gemäß einem Ausführungsbeispiel der Erfindung gezeigt, welches auch eine der in FIG 3 gezeigten Anordnung entsprechende Detektorschaltung 30 umfasst. Das CT-System 40 besteht dabei im Wesentlichen aus einer üblichen Scaneinheit 17, in welcher an einer Gantry 11 eine Projektionsdatenakquisitionseinheit 70 mit einem Detektor 16 und einer dem Detektor 16 gegenüberliegenden Röntgenquelle 15 um einen Messraum 12 umläuft. Vor der Scaneinheit 17 befindet sich eine Patientenlagerungseinrichtung 60 bzw. ein Patiententisch 60, dessen oberer Teil 50 mit einem darauf befindlichen Patienten O zu der Scaneinheit 17 verschoben werden kann, um den Patienten O durch den Messraum 12 hindurch relativ zum Detektorsystem 16 zu bewegen. Angesteuert werden die Scaneinheit 17 und der Patiententisch 60 durch eine Steuereinrichtung 200, von der aus über eine übliche Steuerschnittstelle 24 Akquisitionssteuersignale AS kommen, um das gesamte System gemäß vorgegebener Messprotokolle in der herkömmlichen Weise anzusteuern. Im Fall einer Spiralakquisition ergibt sich durch eine Bewegung des Patienten O entlang der z-Richtung, welche der Systemachse z längs durch den Messraum 12 entspricht, und den gleichzeitigen Umlauf der Röntgenquelle 15 für die Röntgenquelle 15 relativ zum Patienten O während der Messung eine Helixbahn. Parallel läuft dabei immer gegenüber der Röntgenquelle 15 der Detektor 16 mit, um Projektionsmessdaten PMD zu erfassen, die dann zur Rekonstruktion von Volumen- und/oder Schicht-Bilddaten genutzt werden. Ebenso kann auch ein sequentielles Messverfahren durchgeführt werden, bei dem eine feste Position in z-Richtung angefahren wird und dann während eines Umlaufs, eines Teilumlaufs oder mehrerer Umläufe an der betreffenden z-Position die erforderlichen Projektionsmessdaten PMD erfasst werden, um ein Schnittbild an dieser z-Position zu rekonstruieren oder um aus den Projektionsmessdaten mehrerer z-Positionen Bilddaten BD zu rekonstruieren. Das erfindungsgemäße Verfahren und die erfindungsgemäße integrierte Schaltung sind grundsätzlich auch an anderen CT-Systemen, z. B. mit mehreren Röntgenquellen und/oder Detektoren und/oder mit einem einen vollständigen Ring bildenden Detektor, einsetzbar.

Die vom Detektor 16 akquirierten Projektionsmessdaten PMD (im Folgenden auch Rohdaten genannt) werden über eine Rohdatenschnittstelle 23, auch Rohdatenerfassungseinheit genannt, an die Steuereinrichtung 200 übergeben. Diese Rohdaten PMD werden dann, gegebenenfalls nach einer geeigneten Vorverarbeitung (z. B. Filterung und/oder Strahlaufhärtungskorrektur), in einer Bildrekonstruktionseinheit 25 weiterverarbeitet, die in diesem Ausführungsbeispiel in der Steuereinrichtung 200 in Form von Software auf einem Prozessor realisiert ist. Diese Bildrekonstruktionseinheit 25 rekonstruiert auf Basis der Rohdaten PMD Bilddaten BD mit Hilfe eines Rekonstruktionsverfahrens. Als Rekonstruktionsverfahren kann zum Beispiel ein auf der gefilterten Rückprojektion basierendes Rekonstruktionsverfahren verwendet werden.

In die Projektionsmessdaten PMD sind typischerweise auch Statusmeldungen des Detektors 16 integriert. Der Zustand der Überwachungsdurchkontaktierungen aller Detektorschaltkreise kann mit Hilfe dieser Statusmeldungen übermittelt werden. Falls defekte Durchkontaktierungen detektiert werden, wird über die Statusmeldungen eine Fehlermeldung ausgegeben oder gleich ein Serviceeinsatz bestellt.

Die erfassten Bilddaten BD werden in einem Speicher 22 der Steuereinrichtung 200 hinterlegt und/oder in üblicher Weise auf dem Bildschirm der Steuereinrichtung 200 ausgegeben. Sie können auch über eine in FIG 4 nicht dargestellte Schnittstelle in ein an das Computertomographiesystem 40 angeschlossenes Netz, beispielsweise ein radiologisches Informationssystem (RIS), eingespeist und in einem dort zugänglichen Massenspeicher hinterlegt oder auf dort angeschlossenen Druckern oder Filming-Stationen als Bilder ausgegeben werden. Die Daten können so in beliebiger Weise weiterverarbeitet und dann gespeichert oder ausgegeben werden.

Der Detektor 16 umfasst Detektorschaltungen 30, wie sie im Zusammenhang mit FIG 3 beschrieben wurden.

Es wird abschließend noch einmal darauf hingewiesen, dass es sich bei den vorbeschriebenen Verfahren und Vorrichtungen, insbesondere den beschriebenen integrierten Schaltungen und dem beschriebenen Computertomographiesystem, lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Wie bereits erwähnt, kann die Erfindung neben Detektorschaltungen auch auf andere integrierte Schaltungen, wie zum Beispiel Kamera-Sensorschaltkreise oder Speicherschaltkreise, angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Integrierte Schaltung (30), vorzugsweise Detektorschaltung, mit einer Leckstrom-Überwachungsfunktion für Durchkontaktierungen (1) im Substrat der integrierten Schaltung (30), aufweisend:
- eine aktive Schaltung (4),
- einen Schaltungsanschluss (6), welcher mit der aktiven Schaltung (4) über eine Durchkontaktierung (1) elektrisch verbunden ist,
- einen offenen Signalkontakt (6a),
- eine ausschließlich Überwachungszwecken dienende Überwachungsdurchkontaktierung (1a),
- eine Messschaltung (7) mit einem Messeingang (7a) und einem Referenzspannungseingang (7b), wobei der offene Signalkontakt (6a) über die Überwachungsdurchkontaktierung (1a) mit dem Messeingang (7a) der Messschaltung (7) elektrisch verbunden ist,
- einen Betriebsspannungskontakt (U_{B}), welcher über einen Vorwiderstand (RV) elektrisch mit dem Messeingang (7a) verbunden ist, zur Spannungsversorgung der integrierten Schaltung (30).

2. Integrierte Schaltung nach Anspruch 1, aufweisend eine Mehrzahl von parallel geschalteten Überwachungsdurchkontaktierungen, welche durch eine gemeinsame Messschaltung überwacht werden.

3. Integrierte Schaltung nach Anspruch 1 oder 2, wobei der Betriebsspannungskontakt (U_{B}) direkt mit dem Referenzspannungseingang (7b) der Messschaltung (7) verbunden ist.

4. Integrierte Schaltung nach Anspruch 1 oder 2, wobei der Referenzspannungseingang (7b) der Messschaltung (7) mit einem separaten Referenzspannungskontakt elektrisch verbunden ist.

5. Integrierte Schaltung nach einem der vorstehenden Ansprüche, wobei die Messschaltung (7) dazu eingerichtet ist, eine an dem Messeingang (7) anliegende elektrische Messspannung (U_{M}) mit einer Referenzspannung (U_{B}) zu vergleichen und ein Vergleichsergebnis (VE) auszugeben.

6. Integrierte Schaltung nach einem der vorstehenden Ansprüche, welche dazu eingerichtet ist, auf Basis des Vergleichsergebnisses zu ermitteln, ob die Überwachungsdurchkontaktierung (1a) defekt ist.

7. Integrierte Schaltung nach Anspruch 5 oder 6, wobei die Messschaltung (7) dazu eingerichtet ist, eine Spannungsdifferenz zwischen der Referenzspannung (U_{B}) und der Messspannung (U_{M}) zu ermitteln.

8. Integrierte Schaltung nach Anspruch 7, wobei die integrierte Schaltung dazu eingerichtet ist, auf Basis davon, ob die ermittelte Spannungsdifferenz einen Schwellwert überschritten hat, zu ermitteln, ob die Überwachungsdurchkontaktierung (1a) defekt ist.

9. Integrierte Schaltung nach einem der vorstehenden Ansprüche, aufweisend eine Mehrzahl von Überwachungsdurchkontaktierungen (1a), welche über die integrierte Schaltung verteilt sind.

10. Röntgendetektor (16), aufweisend:
- ein Substrat (3),
- eine Sensorschicht auf dem Substrat (3),
- eine integrierte Schaltung (30) nach einem der Ansprüche 1 bis 9.

11. Röntgensystem (40), aufweisend:
- eine Scaneinheit (17) mit mindestens einem Detektor (16) mit einer Mehrzahl von Röntgendetektoreinheiten,
- eine Steuereinrichtung (200) zum Ansteuern der Scaneinheit (17),
wobei mindestens eine der Röntgendetektoreinheiten eine integrierte Schaltung (30) nach einem der Ansprüche 1 bis 9 umfasst.

12. Verfahren zum Überwachen von Leckströmen in einer integrierten Schaltung nach einem der Ansprüche 1 bis 9, aufweisend die Schritte:
- Messen einer an dem Messeingang (7a) der Messschaltung (7) anliegenden Messspannung (U_{M}),
- Messen einer an dem Referenzspannungseingang (7b) anliegenden Referenzspannung (U_{B}),
- Durchführen eines Vergleichs auf Basis der beiden gemessenen Spannungswerte (U_{M}, U_{B}),
- Ermitteln, ob die Überwachungsdurchkontaktierung (1a) defekt ist, auf Basis des Vergleichs.

13. Computerprogrammprodukt mit einem Computerprogramm, welches direkt in eine Speichereinrichtung eines Röntgensystems (40) ladbar ist, mit Programmabschnitten, um alle Schritte des Verfahrens nach Anspruch 12 auszuführen, wenn das Computerprogramm in dem Röntgensystem ausgeführt wird.

14. Computerlesbares Medium, auf welchem von einer Rechnereinheit einlesbare und ausführbare Programmabschnitte gespeichert sind, um alle Schritte des Verfahrens nach Anspruch 12 auszuführen, wenn die Programmabschnitte von der Rechnereinheit ausgeführt werden.

## Claims

1. Integrated circuit (30), preferably detector circuit, with a leakage current monitoring function for through-connections (1) in the substrate of the integrated circuit (30),
having:
- an active circuit (4),
- a circuit connection (6), which is electrically connected to the active circuit (4) by way of a through-connection (1),
- an open signal contact (6a),
- a monitoring through-connection (1a) serving exclusively for monitoring purposes,
- a measuring circuit (7) with a measuring input (7a) and a reference voltage input (7b), wherein the open signal contact (6a) is electrically connected to the measuring input (7a) of the measuring circuit (7) by way of the monitoring through-connection (1a),
- an operating voltage contact (*U_{B}*), which is electrically connected to the measuring input (7a) by way of a preresistor (RV), for the supply of voltage to the integrated circuit (30).

2. Integrated circuit according to claim 1, having a plurality of monitoring through-connections connected in parallel, which are monitored by a shared measuring circuit.

3. Integrated circuit according to claim 1 or 2, wherein the operating voltage contact (*U_{B}*) is connected directly with the reference voltage input (7b) of the measuring circuit (7).

4. Integrated circuit according to claim 1 or 2, wherein the reference voltage input (7b) of the measuring circuit (7) is electrically connected to a separate reference voltage contact.

5. Integrated circuit according to one of the preceding claims, wherein the measuring circuit (7) is designed to compare an electrical measuring voltage (*U_{M}*) present at the measuring input (7) with a reference voltage (*U_{B}*) and to output a comparison result (VE).

6. Integrated circuit according to one of the preceding claims, which is designed to determine, on the basis of the comparison result, whether the monitoring through-connection (1a) is faulty.

7. Integrated circuit according to claim 5 or 6, wherein the measuring circuit (7) is designed to determine a voltage difference between the reference voltage (*U_{B}*) and the measuring voltage (*U_{M}*).

8. Integrated circuit according to claim 7, wherein the integrated circuit is designed to determine, on the basis of whether the determined voltage difference has exceeded a threshold value, whether the monitoring through-connection (1a) is faulty.

9. Integrated circuit according to one of the preceding claims, having a plurality of monitoring through-connections (1a) which are distributed by way of the integrated circuit.

10. X-ray detector (16), having:
- a substrate (3),
- a sensor layer on the substrate (3),
- an integrated circuit (30) according to one of claims 1 to 9.

11. X-ray system (40), having:
- a scanning unit (17) with at least one detector (16) with a plurality of x-ray detector units,
- a control device (200) for actuating the scanning unit (17), wherein at least one of the x-ray detector units comprises an integrated circuit (30) according to one of claims 1 to 9.

12. Method for monitoring leakage currents in an integrated circuit according to one of claims 1 to 9, having the steps:
- measuring a measuring voltage (*U_{M}*) present at the measuring input (7a) of the measuring circuit (7),
- measuring a reference voltage (*U_{B}*) present at the reference voltage input (7b),
- carrying out a comparison on the basis of the two measured voltage values (*U_{M}*, *U_{B}*),
- determining whether the monitoring through-connection (1a) is faulty, on the basis of the comparison.

13. Computer program product with a computer program which can be loaded directly into a storage facility of an x-ray system (40), with program sections in order to carry out all steps of the method according to claim 12, when the computer program is executed in the x-ray system.

14. Computer-readable medium, on which program sections which can be read in and executed by a computing unit are stored, in order to execute all steps of the method according to claim 12, when the program sections are executed by the computing unit.

## Revendications

1. Circuit (30) intégré, de préférence circuit détecteur, ayant une fonction de contrôle de courant de fuite de traversées (1) du substrat du circuit (30) intégré, comportant :
- un circuit (4) actif,
- une borne (6) de circuit, qui est connectée électriquement au circuit (4) actif par une traversée (1),
- un contact (6a) de signal ouvert,
- une traversée (1a) de contrôle servant exclusivement à des fins de contrôle,
- un circuit (7) de mesure, ayant une entrée (7a) de mesure et une entrée (7b) de tension de référence, le contact (6a) de signal ouvert étant connecté électriquement à l'entrée (7a) de mesure du circuit (7) de mesure par la traversée (1a) de contrôle,
- un contact (U_{B}) de tension de fonctionnement, qui est connecté électriquement à l'entrée (7a) de mesure par une résistance (RV) série, pour l'alimentation en tension du circuit (30) intégré.

2. Circuit intégré suivant la revendication 1, comportant une pluralité de traversées de contrôle, qui sont montées en parallèle et qui sont contrôlées par un circuit de mesure commun.

3. Circuit intégré suivant la revendication 1 ou 2, dans lequel le contact (U_{B}) de tension de fonctionnement est connecté directement à l'entrée (7b) de tension de référence du circuit (7) de mesure.

4. Circuit intégré suivant la revendication 1 ou 2, dans lequel l'entrée (7b) de tension de référence du circuit (7) de mesure est connectée électriquement à un contact de tension de référence distinct.

5. Circuit intégré suivant l'une des revendications précédentes, le circuit (7) de mesure étant conçu pour comparer une tension (U_{M}) électrique de mesure s'appliquant à l'entrée (7) de mesure à une tension (U_{B}) de référence et pour émettre un résultat (VE) de comparaison.

6. Circuit intégré suivant l'une des revendications précédentes, qui est conçu pour, sur la base du résultat de comparaison, déterminer si la traversée (1a) de contrôle est défectueuse.

7. Circuit intégré suivant la revendication 5 ou 6, dans lequel le circuit (7) de mesure est conçu pour déterminer une différence de tension entre la tension (U_{B}) de référence et la tension (U_{M}) de mesure.

8. Circuit intégré suivant la revendication 7, dans lequel le circuit intégré est conçu pour, sur la base du point de savoir si la différence de tension déterminée a dépassé une valeur de seuil, déterminer si la traversée (1a) de contrôle est défectueuse.

9. Circuit intégré suivant l'une des revendications précédentes, comportant une pluralité de traversées (1a) de contrôle, qui sont réparties sur le circuit intégré.

10. Détecteur (16) de rayons X, comportant :
- un substrat (3),
- une couche formant capteur sur le substrat (3),
- un circuit (30) intégré suivant l'une des revendications 1 à 9.

11. Système (40) à rayons X, comportant :
- une unité (17) de balayage, ayant au moins un détecteur (16), ayant une pluralité d'unités de détecteur de rayons X,
- un dispositif (200) de commande, pour commander l'unité (17) de balayage,
dans lequel au moins l'une des unités de détecteur de rayons X comprend un circuit (30) intégré suivant l'une des revendications 1 à 9.

12. Procédé de contrôle de courant de fuite d'un circuit intégré suivant l'une des revendication 1 à 9, comportant les stades :
- on mesure une tension (U_{M}) de mesure s'appliquant à l'entrée (7a) de mesure du circuit (7) de mesure,
- on mesure une tension (U_{B}) de référence s'appliquant à l'entrée (7b) de tension de référence,
- on effectue une comparaison sur la base des deux valeurs (U_{M}, U_{B}) de tension, qui ont été mesurées,
- on détermine si la traversée (1a) de contrôle est défectueuse, sur la base de la comparaison.

13. Produit de programme d'ordinateur, ayant un programme d'ordinateur qui peut être chargé directement dans un dispositif de mémoire d'un système (40) à rayons X, comprenant des parties de programme pour effectuer tous les stades du procédé suivant la revendication 12, lorsque le programme d'ordinateur est réalisé dans le système à rayons X.

14. Support déchiffrable par ordinateur, sur lequel sont mémorisées des parties de programme pouvant être lues et réalisées par une unité d'ordinateur, pour effectuer tous les stades du procédé suivant la revendication 12, lorsque les parties de programme sont réalisées par l'unité d'ordinateur.
